# EUROPÄISCHE PATENTSCHRIFT

(11) **EP 4 631 463 B1**
(45) Veröffentlichungstag und Bekanntmachung des Hinweises auf die Patenterteilung: **04.11.2026**
(21) Anmeldenummer: 25170101.7
(22) Anmeldetag: 11.04.2025
(51) Int. Cl.: A61B 42/40, A47K 10/18

(54) **VORRICHTUNG ZUM HALTEN UND BEREITSTELLEN EINER STAPELFÖRMIGEN ANORDNUNG VON FLACHMATERIALABSCHNITTEN, INSBESONDERE VON REINIGUNGS- ODER DESINFEKTIONSTÜCHERN, ODER VON FLACHGELEGTEN EINMAL-HANDSCHUHEN**
DEVICE FOR HOLDING AND PRESENTING A STACK-SHAPED ARRANGEMENT OF FLAT MATERIAL SECTIONS, IN PARTICULAR OF CLEANING OR DISINFECTING WIPES, OR OF FLAT DISPOSABLE GLOVES
DISPOSITIF POUR TENIR ET FOURNIR UN ARRANGEMENT EN FORME D'EMPILEMENT DE SECTIONS DE MATÉRIAU PLAT, EN PARTICULIER DE LINGETTES NETTOYANTES OU DÉSINFECTANTES, OU DE GANTS JETABLES APLATIS

(30) Priorität: 11.04.2024 DE 102024110142
(43) Veröffentlichungstag der Anmeldung: 15.10.2025
(73) Patentinhaber: Bode Chemie GmbH, 22525 Hamburg (DE)
(72) Erfinder: Langlotz, Christian, 22525 Hamburg (DE)
(74) Vertreter: DREISS Patentanwälte PartG mbB

(56) Entgegenhaltungen:
- NL-C2- 2 006 341
- US-A1- 2014 263 396

## Beschreibung

Die vorliegende Erfindung betrifft eine Vorrichtung, insbesondere zur Verwendung in Gesundheitseinrichtungen, zum Halten und Bereitstellen einer insbesondere umverpackten stapelförmigen Anordnung von Flachmaterialabschnitten, insbesondere von Reinigungs- oder Desinfektionstüchern, oder von flachgelegten Einmal-Handschuhen, damit der Endverbraucher aufeinanderfolgend einzelne Flachmaterialabschnitte oder Handschuhe aus der stapelförmigen Anordnung entnehmen kann,
- wobei die Flachmaterialabschnitte oder Handschuhe jeweils in einer Stapelebene erstreckt sind und orthogonal zu dieser Stapelebene mittelbar oder unmittelbar aneinander anliegen,
- wobei die Vorrichtung ein hinteres Stützmittel und ein vorderes Stützmittel aufweist, wobei das vordere Stützmittel bei bestimmungsgemäßer Positionierung und Verwendung der Vorrichtung dem Endverbraucher zugewandt ist und eine Öffnung oder Ausnehmung für den Zugriff auf die stapelförmige Anordnung durch den Endverbraucher aufweist,
- wobei die Vorrichtung bei bestimmungsgemäßer Positionierung und Verwendung der Vorrichtung derart ausgebildet ist, dass die stapelförmige Anordnung darin mit im Wesentlichen vertikal orientierter Stapelebene aufnehmbar ist.

Professionelle Hygienemaßnahmen sind unumstritten ein Weg zur Verhinderung von Übertragungen mikrobieller Pathogene im Gesundheitswesen, insbesondere in Kranken- oder Pflegeeinrichtungen. Hierbei spielt der Einsatz von Reinigungs- oder Desinfektionstüchern ebenso wie die konsequente Verwendung von Einmal-Handschuhen eine große Rolle. Auch die Art der Ausführung des Reinigungs- oder Desinfektionsvorgangs hat einen entscheidenden Einfluss auf die Effektivität der Reinigung oder Desinfektion.

Daneben kommt aber der Compliance in Bezug auf das Reinigungs- oder Desinfektionsverhalten ebenso wie der Bereitschaft, Einmal-Handschuhe zu tragen und zu wechseln, eine ebenso herausragende Bedeutung zu. Mithin ist das Verhältnis von einer Anzahl von tatsächlich erfolgten Reinigungen oder Desinfektionen bzw. Verwendungen von Einmal-Handschuhen zu der Anzahl an Gelegenheiten, bei denen eine Reinigung oder Desinfektion bzw. die Verwendung von Einmal-Handschuhen unter hygienischen Gesichtspunkten erfolgen sollte, wesentlich.

Die Gelegenheiten, bei denen eine Reinigung oder Desinfektion erfolgen sollte oder bei denen Einmal-Handschuhe getragen werden sollten, stehen häufig in engem Zusammenhang mit der Behandlung oder Pflege von Patienten durch das pflegende oder behandelnde Personal. Es hat sich hier gezeigt, dass die Compliance verbessert werden kann, wenn die Verfügbarkeit von Reinigungs- oder Desinfektionstüchern bzw. von Einmal-Handschuhen in der Nähe oder besser unmittelbar am sogenannten "Point-of-Care", mithin unmittelbar dort, wo die Behandlung oder Pflege, also der Patientenkontakt, erfolgt, gegeben ist.

Es sind daher bereits Vorrichtungen und Systeme bekannt, die die Bereitstellung von Packungen von Reinigungstüchern, Desinfektionstüchern oder Einmal-Handschuhen am oder in der Nähe des Point of Care ermöglichen.

EP 2 915 471 A1 beschreibt eine Halterung für eine Tücherpackung. Die Halterung umfasst eine Auflageplatte, seitlich angeordnete Platten und eine rückseitig vorhandene Platte mit einem Befestigungsbereich. DE 20 2010 004 469 U1, US 5,839,632, US 2007/290108 A1, US 3,837,608,
US 2016/278518 A1 und US 2004/099623 A1 offenbaren andere Haltevorrichtungen und Haltesysteme. Reinigungstücher, Desinfektionstücher und Einmal-Handschuhe werden üblicherweise in Folienbeuteln oder in Verpackungen aus einem Pappmaterial bereitgestellt, wobei die Verpackung eine gegebenenfalls mit einem Deckel verschließbare Entnahmeöffnung aufweist.

Reinigungstücher oder Desinfektionstücher liegen in der Verpackung häufig als stapelförmige Anordnung, und zwar zumeist als gefalteter Stapel vor, wobei die einzelnen Tücher einander zumeist ganz oder auch nur teilweise überlappen und insbesondere teilweise ineinander eingreifen, wobei die Faltung meist so erfolgt, dass beim Herausziehen eines aus der Entnahmeöffnung herausragenden Tuchs das nachfolgende Tuch etwas mitgenommen wird, so dass dieses dann in der Entnahmeöffnung für einen Nutzer als nächstes greifbar bereitgestellt wird. Die Ablösung des herausgezogenen Tuchs von dem nachfolgenden Tuch wird durch die Reibung des nachfolgenden Tuchs an der Innenseite der Verpackung unterstützt. Ein ähnlicher Effekt erleichtert einem Nutzer auch das Herausnehmen von Einmal-Handschuhen aus einer Umverpackung, wobei in diesem Fall die Handschuhe zumeist aufgrund von Adhäsion trennbar aneinanderhaften.

Bei der Entnahme von Tüchern oder Handschuhen aus der Verpackung kann sich das Problem ergeben, dass der bei einer zunehmenden Entleerung der Verpackung entstehende Hohlraum zwischen den verbleibenden Tüchern oder Handschuhen und der Entnahmeöffnung das weitere Herausnehmen von Tüchern oder Handschuhen erschwert. Zum einen lässt nämlich die oben geschilderte Friktion zwischen dem Produkt und der Verpackung nach, da bei einem kleiner werdenden Füllstand die von den Artikeln auf die Verpackung ausgeübte Spannung zurückgeht. Dies kann dazu führen, dass der Nutzer beim Herausziehen eines in der Entnahmeöffnung vorhandenen Artikels eine Vielzahl weiterer Artikel nachzieht, da sich diese nicht wie vorgesehen voneinander lösen. Zum anderen muss der Nutzer, wenn sich kein Artikel greifbar im Bereich der Entnahmeöffnung befindet, tiefer in die Verpackung hineingreifen, um den Artikel, also das Reinigungstuch, das Desinfektionstuch oder den Einmal-Handschuh, herausnehmen zu können.

EP 2 974 980 A1 offenbart einen Tuchspender, bei dem die Verpackung ein im Bereich der Entnahmeöffnung vorhandenes und nach innen drückendes Element aufweist. Das Element kann einen Druck auf die Oberseite des Tücherstapels ausüben, um die bei einer zunehmenden Entleerung der Verpackung reduzierte Spannung des Stapels auf die Innenseite der Verpackung zu kompensieren. EP 3 629 864 B1 zeigt einen an einem Patientenbett befestigbaren Tuchspender zum Halten und Bereitstellen einer stapelförmigen Anordnung von Flachmaterialabschnitten mit vertikaler Stapelebene, bei dem die Tücher mittels eines Federelements in horizontaler Richtung orthogonal zu ihrer Stapelebene vorgespannt sind.

NL 2 006 341 C2 offenbart eine gattungsgemäße Haltevorrichtung für eine stapelförmige Anordnung von Flachmaterialabschnitten, in einer Nachfüllpackung, wobei die Nachfüllpackung mit einer Entnahmeöffnung in einer stets gleichen bestimmungsgemäßen Position an der Haltevorrichtung für die Entnahme der Flachmaterialabschnitte angeordnet wird. Eine entsprechende ähnliche Haltevorrichtung ist in US 2014/0263396 A1 offenbart.

Bei einer Bereitstellung einer Verpackung mit feuchten Reinigungstüchern oder Desinfektionstüchern am "Point-of-care" mit horizontaler Stapelebene und einer nach oben zeigenden Entnahmeöffnung ergibt sich häufig das weitere Problem, dass die in der Verpackung vorhandene Flüssigkeit zum Boden der Verpackung sickert, mit der Folge, dass die weiter oben im Stapel vorhandenen Tücher nicht ausreichend mit der Reinigungs- oder Desinfektionslösung getränkt sind. Um diesem Problem zu begegnen, wäre es vorteilhaft, die Verpackung am "Point-of-care" in einer vertikalen Ausrichtung der Stapelebene und mit einer zur Seite hinzeigenden Entnahmeöffnung bereitzustellen.

Die bekannten Lösungen haben sich zudem hinsichtlich der Positionierbarkeit der Packungen am "Point-of-care" als verbesserungswürdig erwiesen. Im Sinne der Compliance ist es weiterhin wünschenswert, dass die Packungen mühelos in die Haltevorrichtung eingebracht werden können und danach in einer Anwendungssituation sicher gehalten sind.

Ansprüche 1, 23 und 24 definieren die Erfindung. Abhängige Ansprüche offenbaren Ausführungsformen. Der vorliegenden Erfindung liegt die Aufgabe zugrunde, die Handhabbarkeit bekannter Vorrichtungen und Systeme zu verbessern. Insbesondere soll eine Verbesserung hinsichtlich einer einfachen Befüllbarkeit der Vorrichtung mit insbesondere umverpackten Flachmaterialabschnitten oder flachgelegten Einmal-Handschuhen sowie hinsichtlich einer als angenehm empfundenen Entnehmbarkeit dieser Produkte erzielt werden, was die Bereitschaft zur Benutzung erhöht und damit der Hygiene förderlich ist.

Diese Aufgabe wird bei einer Vorrichtung der eingangs genannten Art erfindungsgemäß dadurch gelöst, dass das vordere Stützmittel und das hintere Stützmittel bei bestimmungsgemäßer Positionierung und Verwendung der Vorrichtung eine sich nach vertikal unten verjüngende und nach vertikal oben erweiternde Keilform für die Aufnahme der insbesondere umverpackten stapelförmigen Anordnung begrenzen, so dass die insbesondere umverpackte stapelförmige Anordnung mit zunehmender Anzahl der bereits entnommenen Flachmaterialabschnitte oder Handschuhe und damit korrelierend mit abnehmender verbleibender Dicke der stapelförmigen Anordnung in der Vorrichtung schwerkraftbedingt weiter nach vertikal unten gleiten kann, so dass infolge der Keilform durch das hintere und das vordere Stützmittel stets ein quer zur Stapelebene wirkender Stützdruck ungefähr in horizontaler Richtung auf die stapelförmige Anordnung ausgeübt wird, und dass das vordere Stützmittel und das hintere Stützmittel bereichsweise einen die Keilform begrenzenden Winkel (α) von wenigstens 20° einschließen.

Mit der vorliegenden Erfindung wird also vorgeschlagen, die Form der Vorrichtung durch Ausbildung der beschriebenen Keilform durch das vordere und hintere Stützmittel dafür einzusetzen, dass die stapelförmige Anordnung auch bei abnehmender Stapeldicke stets selbsttätig und schwerkraftbedingt in eine stabile Stützlage gelangt, so dass der als nächstes zu entnehmende Flachmaterialabschnitt oder Handschuh unter moderatem Stützdruck nach vorn in den Bereich der Entnahmeöffnung positioniert wird, ohne dass hierfür separate Federmittel oder dergleichen verwendet werden müssten. Dies geschieht nach der Erfindung vielmehr allein durch die auf die stapelförmige Anordnung wirkende Schwerkraft innerhalb der Vorrichtung. Die Schwerkraft im Zusammenwirken mit der beschriebenen Keilform führt zu einem quer zur Stapelebene wirkenden moderaten Stützdruck auf die stapelförmige Anordnung, wodurch die Ausbildung von Hohlräumen innerhalb einer Umverpackung der stapelförmige Anordnung vermieden wird und der Benutzer zum Entnehmen eines weiteren Flachmaterialabschnitts oder Handschuhs nicht tief in die Verpackung eingreifen muss, was wiederum die Gefahr von Kontamination der darin enthaltenen Produkte mit sich bringen würde.

Ein weiterer Vorteil, der durch die beschriebene Keilform realisiert wird, ist darin zu sehen, dass verschieden dicke stapelförmige Anordnungen in ein und derselben Haltevorrichtung aufnehmbar sind; sie akkommodieren sich selbsttätig innerhalb der Keilform, was bei Haltevorrichtungen mit einem zusätzlichen Federelement nicht ohne weiteres der Fall ist, weil das Federelement je nach Stapeldicke zu stark oder zu schwach ist, um ein komfortables Entnehmen des Inhalts stets zu gewährleisten. Auch würde durch ein solches zusätzliches Federelement die Komplexität der Haltevorrichtung und damit der Kostenaufwand weiter erhöht. Ein weiterer Nachteil von zusätzlichen Federelementen ist, dass mit abnehmender Dicke der stapelförmigen Anordnung die Produkte durch die vordere Entnahmeöffnung hindurchgedrückt werden können und dabei insbesondere eine wiederverschließbare Öffnung bei der Verpackung geöffnet oder sogar beschädigt wird und die darin enthaltenen Produkte austrocknen.

Wenn die stapelförmige Anordnung umverpackt ist, also sich insbesondere und typischerweise in einer Schlauchbeutel-Packung befindet, dann sollte diese Umverpackung aus einem biegeschlaffen Material und jedenfalls nicht aus einem biegesteifen Material gebildet sein, damit die stapelförmige Anordnung samt Umverpackung mit abnehmender Dicke in der Vorrichtung zwischen dem vorderen und dem hinteren Stützmittel in vertikaler Richtung nach unten gleiten kann.

Zur Realisierung der erfindungsgemäßen Vorrichtung erweist es sich als vorteilhaft, dass das vordere Stützmittel und/oder das hintere Stützmittel flächenhaft wirkend ausgebildet ist und sich über wenigstens 50 %, insbesondere über wenigstens 60 %, insbesondere über wenigstens 70 %, insbesondere über wenigstens 80 % der flächenhaften Erstreckung der stapelförmigen Anordnung in der Stapelebene und weiter insbesondere über die gesamte flächenhafte Erstreckung der stapelförmigen Anordnung in der Stapelebene erstreckt. Hierbei wird bei der Ermittlung der flächenhaften Erstreckung des vorderen Stützmittels die Fläche einer Öffnung oder Ausnehmung für die Entnahme nicht berücksichtigt, also nicht in Abzug gebracht.

Es erweist sich als vorteilhaft und einfach, wenn das hintere Stützmittel und/oder das vordere Stützmittel insbesondere von einem flächenhaft durchgehenden Material gebildet ist. Insbesondere erweist es sich als vorteilhaft, wenn das hintere Stützmittel und/oder das vordere Stützmittel von einer hinteren bzw. vorderen Stützwand gebildet sind. Insbesondere erweist es sich als vorteilhaft, wenn die Vorrichtung aus einem metallischen Flachmaterial gebogen ist. Insbesondere erweist es sich als vorteilhaft, wenn die Vorrichtung als Kunststoffspritzteil hergestellt ist. All diese weiteren beispielhaften Ausführungen gestatten eine sehr kostengünstige, kompakt anmutende und funktionale Realisierung der erfindungsgemäßen Vorrichtung.

Es ist weiter denkbar, dass die Vorrichtung seitlich einseitig oder beidseitig offen ausgebildet ist, oder dass sie seitlich einseitig oder beidseitig geschlossen ausgebildet ist. Die offene Ausbildung ist mit dem Vorteil einer geringeren Bauform verbunden, was die Verpackung, Lagerhaltung und Lieferung der Vorrichtung kostengünstig beeinflusst und insbesondere die Möglichkeit eröffnet, dieselbe Vorrichtung für verschieden ausladende stapelförmige Anordnungen zu verwenden.

Weiter kann es sich als vorteilhaft erweisen, wenn die Öffnung oder Ausnehmung bei dem vorderen Stützmittel für den Zugriff auf die stapelförmige Anordnung randoffen ausgebildet ist, also nicht als Fenster allseits umschlossen ausgebildet ist, sondern sich ausgehend von einem Außenrand des vorderen Stützmittels nach innen erstreckt. Dies vermittelt ein leichtes Erscheinungsbild und verbessert außerdem die Zugreifbarkeit auf diese Öffnung. Es wäre indessen auch denkbar, die Öffnung oder Ausnehmung als allseits umgebene Fensteröffnung auszubilden, etwa wenn eine höhere Stabilität des vorderen Stützmittels erreicht werden soll.

Wie schon erwähnt, kann bei der erfindungsgemäßen Vorrichtung auf ein weiteres oder zusätzliches federndes Stützmittel ohne weiteres verzichtet werden.

Es wird weiter vorgeschlagen, dass das vordere Stützmittel und das hintere Stützmittel ineinander übergehen, insbesondere einstückig ineinander übergehen, insbesondere verrundet ineinander übergehen. Auch hierdurch wird eine einfache Herstellbarkeit und ein reduziertes zweckmäßiges Design realisiert.

Es wird weiter vorgeschlagen, dass das vordere Stützmittel bei Betrachtung einer vertikalen orthogonal zu dem vorderen und dem hinteren Stützmittel verlaufenden Schnittebene in einer Richtung von unten nach oben gekrümmt verläuft, insbesondere sich ausgehend von der Keilform einem vertikalen Verlauf annähert. Durch gekrümmte Formgebung lässt sich ein gefälliges Design realisieren, und außerdem kann hierdurch die Funktionsweise sowie die Verwindungsstabilität der Vorrichtung verbessert beziehungsweise erhöht werden.

Bei einer bevorzugten Ausführungsform enden oder münden das vordere und das hintere Stützmittel nach oben frei aus und bilden einen oberen Randbereich. Insbesondere wird vorgeschlagen, dass das vordere Stützmittel und das hintere Stützmittel in einem oberen Bereich eine nach oben offene Einführöffnung zum Einsetzen der insbesondere umverpackten stapelförmigen Anordnung in die Vorrichtung bilden. Dieser obere Randbereich bzw. die hierdurch gebildete Einführöffnung kann je nach Ausbildung und Quer- bzw. Umfangserstreckung des vorderen und des hinteren Stützmittels seitlich offen oder in Umfangsrichtung durchgehend ausgebildet sein.

In Weiterführung dieses Gedankens kann es sich als vorteilhaft erweisen, wenn das vordere Stützmittel und das hintere Stützmittel in einem oberen Bereich, welcher eine Einführöffnung für die insbesondere umverpackte stapelförmige Anordnung bildet, zumindest bereichsweise parallel zueinander und bei bestimmungsgemäßer Anordnung und Verwendung vertikal orientiert sind. Hierdurch kann eine zusätzliche Führungsfunktion beim Einsetzen der stapelförmigen Anordnung in die Vorrichtung realisiert werden.

Die Ausbildung und Anordnung des vorderen und des hinteren Stützmittels zueinander ist bei einer generell bevorzugten Ausführungsform der erfindungsgemäßen Haltevorrichtung wie folgt:
In einem oberen Bereich, insbesondere im Bereich einer oberen Öffnung zum Einsetzen der stapelförmigen Anordnung in die Vorrichtung, beträgt eine Tiefenerstreckung der Keilform, also ein Abstand des vorderen Stützmittels vom hinteren Stützmittel in horizontaler Richtung, wenigstens 5 cm, insbesondere wenigstens 6 cm, insbesondere wenigstens 7 cm, insbesondere wenigstens 8 cm und insbesondere höchstens 25 cm, insbesondere höchstens 23 cm, insbesondere höchstens 20 cm, insbesondere höchstens 18 cm, insbesondere höchstens 16 cm. In vertikaler Richtung nach unten gehend verringert sich diese Tiefenerstreckung der Keilform, d.h. der Abstand des vorderen Stützmittels vom hinteren Stützmittel in horizontaler Richtung dann auf höchstens 8 cm, insbesondere auf höchstens 6 cm, insbesondere auf höchstens 4 cm, insbesondere auf höchstens 2 cm. Daher kann die stapelförmige Anordnung schwerkraftbedingt je nach verbleibender Dicke des Stapels nach unten gleiten, so dass sie im unteren Bereich stets zwischen dem vorderen und dem hinteren Stützmittel unter einem moderaten schwerkraftbedingten Stützdruck lagestabilisiert ist.

Zur Ausbildung der erwähnten Keilform erweist es sich weiter als vorteilhaft, wenn das vordere Stützmittel und das hintere Stützmittel bereichsweise einen die Keilform begrenzenden Winkel von wenigstens 25°, insbesondere von wenigstens 30°, insbesondere von wenigstens 35°, insbesondere von höchstens 60°, insbesondere von höchstens 55°, insbesondere von höchstens 50°, insbesondere von höchstens 45°, insbesondere von höchstens 40° einschließen.

Insbesondere für die Anbringung der Vorrichtung an einer vertikalen Wand erweist es sich als vorteilhaft, wenn das hintere Stützmittel bei bestimmungsgemäßer Positionierung und Verwendung der Vorrichtung in einer vertikalen Ebene erstreckt ist.

Weiter kann es sich als zweckmäßig erweisen, dass das hintere Stützmittel ein Montagemittel, insbesondere ein Wandmontagemittel, aufweist, insbesondere Öffnungen, einen Halter oder Haken oder ein Haftklebemittel aufweist, insbesondere einen vorzugsweise werkzeuglos auf die Vorrichtung aufklipsbaren Halter oder Haken aufweist. Diese Montagemittel können beispielsweise zur Montage der Vorrichtung an einer Wand oder auch für eine hängende Befestigung an einem Patientenbett verwendet werden. Ein Halter oder Haken für die Vorrichtung ist vorzugsweise werkzeuglos und insbesondere lösbar an der Vorrichtung befestigbar, so dass er als Zusatz- oder Zubehörteil bereitgestellt werden kann. Als selbstständig erfindungsbegründend wird auch ein solcher werkzeuglos auf die Vorrichtung aufklipsbarer Halter oder Haken angesehen, mittels dessen die Vorrichtung an einem Patientenbett oder an einem Ständer oder an einer sonstigen übergreifbaren Anordnung anhängbar ist und auch wieder abgenommen werden kann. Der Halter oder Haken umfasst hierfür eine in vertikaler Ebene erstreckte Strebe, eine Lasche oder einen Wandabschnitt, welche(r) die Vorrichtung, insbesondere das hintere Stützmittel der Vorrichtung, von oben und von unten ergreift oder umgreift oder in eine Ausnehmung des hinteren Stützmittels eingreift und hierdurch die Vorrichtung zu halten vermag. Hierfür umfasst die Strebe, die Lasche oder der Wandabschnitt in der Gebrauchssituation oben einen Hintergriffsabschnitt, insbesondere einen um vorzugsweise 180° gekröpften Randabschnitt, welcher einen oberen Rand des hinteren Stützmittels von oben formschlüssig übergreift oder in eine Ausnehmung des hinteren Stützmittels eingreift, und einen unteren unter die Vorrichtung greifenden Stützabschnitt, auf welchem die Vorrichtung mit ihrem unteren Ende aufsitzen kann und hierdurch gestützt und gehalten ist. Insbesondere der untere Stützabschnitt der Strebe, der Lasche oder des Wandabschnitts kann auch leicht federnd ausgebildet sein. Des weiteren umfasst der Halter oder Haken einen Einhänge- oder Aufhängeabschnitt, mittels dessen der Halter und die daran gehaltene Vorrichtung samt Inhalt an einem Patientenbett oder an einem Ständer oder an einer sonstigen übergreifbaren Anordnung aufhängbar ist. Der Einhänge- oder Aufhängeabschnitt kann hierfür einen nach unten öffnenden Aufnahmeschlitz begrenzen, der von der in vertikaler Ebene erstreckten Strebe, der Lasche oder dem Wandabschnitt einerseits begrenzt sein kann. Der werkzeuglos auf die Vorrichtung aufklipsbare Halter oder Haken kann demnach eine im wesentlichen S-förmige Grundkontur aufweisen.

Insbesondere erweist sich die erfindungsgemäße Vorrichtung in Verbindung mit einer sogenannten Schlauchbeutel-Packung (Flowpack-Packung) von Flachmaterialabschnitten oder Handschuhen als vorteilhaft.

Gegenstand der Erfindung ist auch ein System aus einer Vorrichtung wie vorausgehend beschrieben und einer insbesondere umverpackten stapelförmigen Anordnung von Flachmaterialabschnitten, insbesondere von Reinigungs- oder Desinfektionstüchern, oder von flachgelegten Einmal-Handschuhen, zur Abgabe der Flachmaterialabschnitte oder Einmal-Handschuhe an Endverbraucher derart, dass ein Endverbraucher aufeinanderfolgend einzelne Flachmaterialabschnitte oder Handschuhe aus der stapelförmigen Anordnung im Wesentlichen in horizontaler Richtung entnehmen kann. Ferner ist Gegenstand der Erfindung eine Verwendung der Vorrichtung nach Anspruch 24.

Weitere Merkmale, Einzelheiten und Vorteile der Erfindung ergeben sich aus den beigefügten Patentansprüchen und aus der zeichnerischen Darstellung und nachfolgenden Beschreibung von Ausführungsformen der erfindungsgemäßen Vorrichtung.

In der Zeichnung zeigt:
Figuren 1a-c verschiedene Ansichten einer ersten Ausführungsform einer erfindungsgemäßen Vorrichtung;
Figuren 2a-c verschiedene Ansichten einer zweiten Ausführungsform einer erfindungsgemäßen Vorrichtung;
Figuren 3a-d verschiedene Ansichten der Vorrichtung nach Figuren 1a-c mit einer darin gehaltenen umverpackten stapelförmigen Anordnung von Flachmaterialabschnitten in befülltem und in zunehmend entleertem Zustand;
Figur 4 die Vorrichtung nach Figuren 1a-c mit einer darin gehaltenen über die offenen Seiten vorstehenden stapelförmigen Anordnung von Flachmaterialabschnitten und
Figuren 5a-c einen auf die erfindungsgemäße Vorrichtung nach Figuren 2a-c aufklipsbaren Halter oder Haken in perspektivischer Darstellung und in der Anbringung an der Vorrichtung.

Die Figuren zeigen Ausführungsformen einer erfindungsgemäßen Vorrichtung 2 zum Halten und Bereitstellen einer im dargestellten Fall umverpackten stapelförmigen Anordnung 4 von Flachmaterialabschnitten 6, insbesondere von Reinigungs- oder Desinfektionstüchern, oder von flachgelegten Einmal-Handschuhen, damit der Endverbraucher aufeinanderfolgend einzelne Flachmaterialabschnitte 6 oder Handschuhe aus der stapelförmigen Anordnung 4 entnehmen kann (nachfolgend als Haltevorrichtung 2 bezeichnet). Bei der Umverpackung 8 der stapelförmigen Anordnung 4 handelt es sich beispielhaft um eine Schlauchbeutel-Packung (Flow-Pack) 10 mit einer im beispielhaft dargestellten Fall wiederverschließbaren Öffnung 12 zum aufeinanderfolgenden Entnehmen von darin stapelförmig und damit parallel zueinander angeordneten Flachmaterialabschnitten 6 oder flachgelegten Handschuhen.

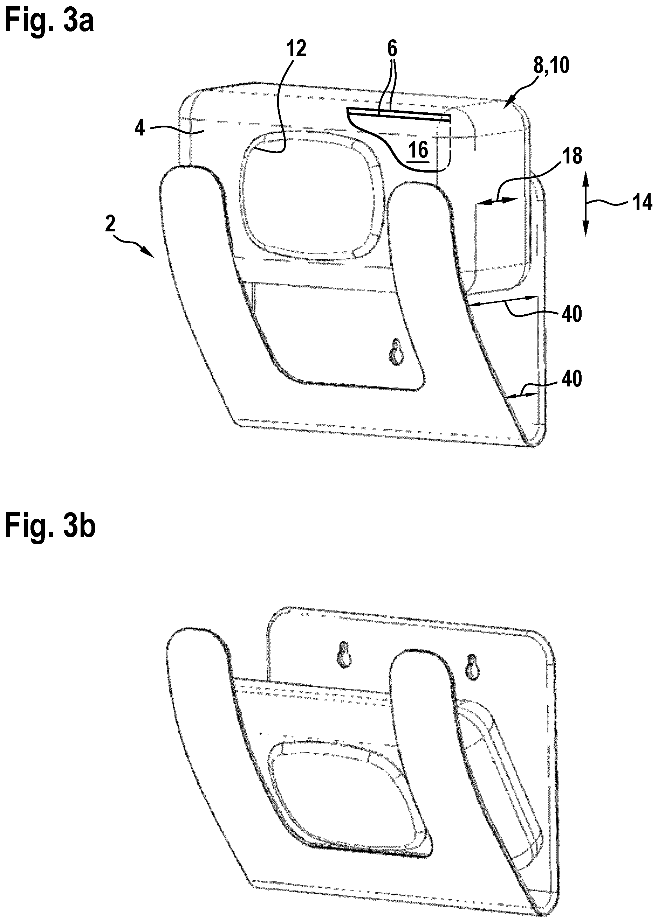

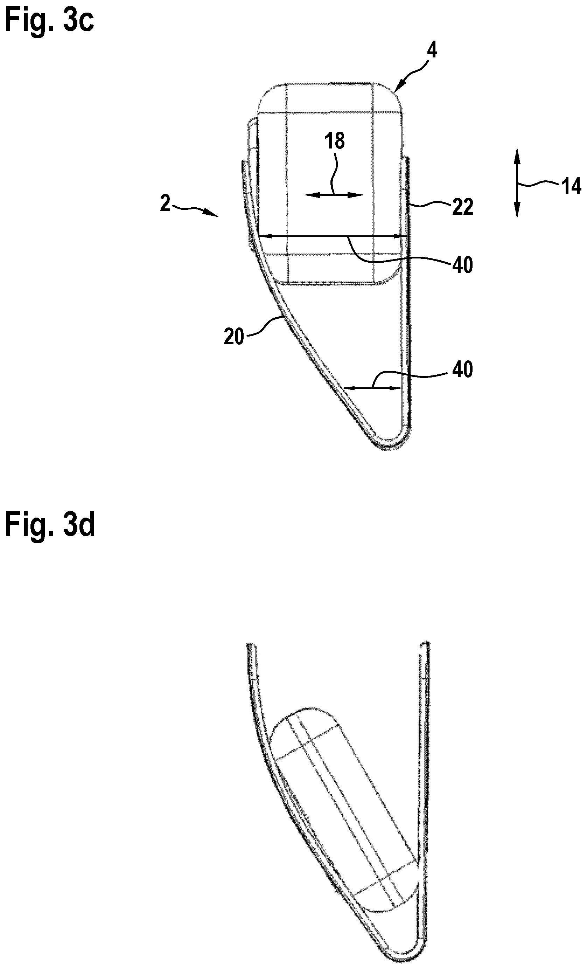

Die nachfolgend im Einzelnen zu beschreibende erfindungsgemäße Haltevorrichtung 2 für die stapelförmige Anordnung 4 ist so ausgebildet, dass die stapelförmige Anordnung 4 und die darin enthaltenen Flachmaterialabschnitte 6 zumindest zu Beginn des Entnahmevorgangs mit ihrer in den Figuren 3a, b angedeuteten Stapelebene 16 im Wesentlichen vertikal orientiert sind. Somit ist auch die Entnahmeöffnung 12 im Wesentlichen in vertikaler Richtung 14 orientiert, und die Flachmaterialabschnitte 6 oder flachgelegte Handschuhe, werden dann orthogonal zu ihrer vertikal orientierten Stapelebene 16, also in horizontaler Richtung 18, aus der Umverpackung 8 entnommen.

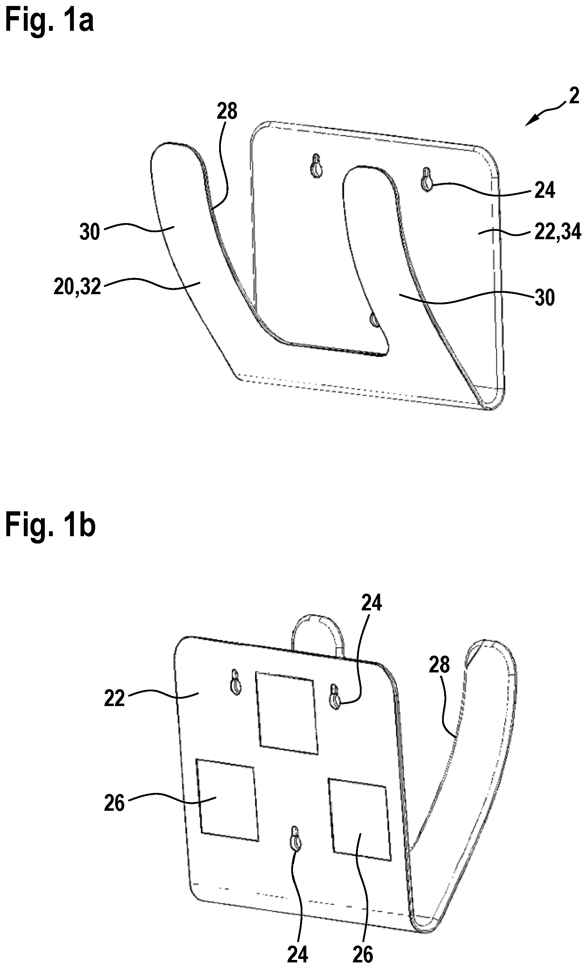

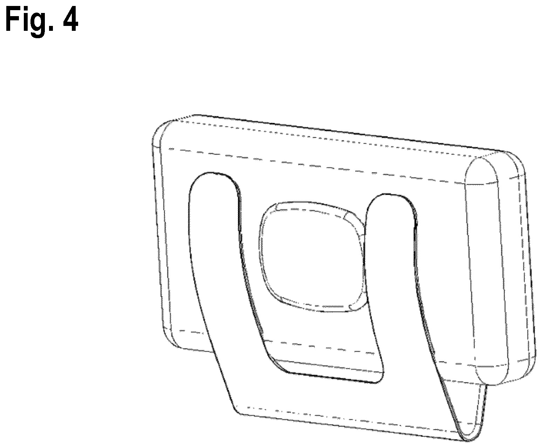

Die erfindungsgemäße Haltevorrichtung 2 ist in einer Ausführungsform in Figuren 1a bis c ohne und in Figuren 3a bis 3d und Figur 4 mit einer darin eingesetzten Schlauchbeutelpackung 10 mit unterschiedlichem Füllstand und daher unterschiedlicher Dicke der stapelförmigen Anordnung 4 von Flachmaterialabschnitten 6 dargestellt.

Die in Figuren 1a bis c dargestellte Haltevorrichtung 2 umfasst ein vorderes Stützmittel 20 und ein hinteres Stützmittel 22, wobei das vordere Stützmittel 20 bei bestimmungsgemäßer Positionierung und Verwendung der Haltevorrichtung 2 einem Endverbraucher zugewandt ist. Das hintere Stützmittel 22 ist zur Anbringung an einer Wand oder auf noch zu beschreibende Weise zum Anhängen an ein Patientenbett oder ein sonstiges Funktionsmöbel oder einen Ständer konzipiert. Im beispielhaft dargestellten Fall sind bei dem hinteren Stützmittel 22 hierfür Montagemittel in Form von Montageöffnungen 24 und ferner Haftklebefelder oder - mittel 26 vorgesehen.

Das vordere Stützmittel 20 und das hintere Stützmittel 22 sind im beispielhaft und bevorzugt dargestellten Fall einstückig miteinander ausgebildet. Es kann sich hierbei beispielsweise um ein einstückiges Kunststoffspritzteil oder bevorzugtermaßen um ein Blechbiegeteil handeln, welches ausgehend von einem ursprünglich ebenen metallischen Flachmaterialabschnitt durch Schneide-/Stanz-/ und/oder Biegevorgänge hergestellt sein kann.

In dem vorderen Stützmittel 20 ist eine nach oben randoffene und ausladend ausgebildete Ausnehmung 28 ausgebildet und beispielhaft durch zwei eher schmal bauende sich nach vertikal oben erstreckende streifenförmige Stützzungen 30 des vorderen Stützmittels 20 begrenzt. Durch die Ausnehmung 28 hindurch kann ein Benutzer auf die stapelförmige Anordnung 4 zugreifen. Das vordere Stützmittel 20 und das hintere Stützmittel 22 bilden im beispielhaft dargestellten Fall eine vordere bzw. hintere Stützwand 32, 34, zwischen denen die Schlauchbeutelpackung 10 wie in den Figuren 3a bis d gezeigt aufgenommen wird. Die vordere Stützwand 32 umfasst im beispielhaft dargestellten Fall die schon erwähnte verhältnismäßig große Ausnehmung 28 zwischen den beiden Stützzungen 30 während die hintere Stützwand 34 bis auf die Montageöffnungen 24 flächenhaft durchgehend ausgebildet ist und daher eine stabile verwindungssteife Montage der Haltevorrichtung 2 gestattet.

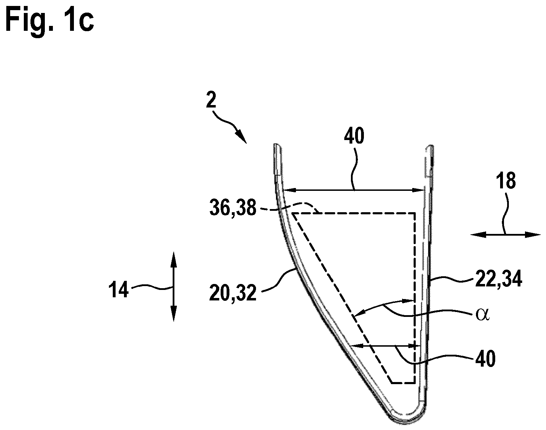

Die Haltevorrichtung 2 und dessen vorderes und hinteres Stützmittel 20, 22 sind erfindungsgemäß so ausgebildet, dass sie eine sich nach vertikal unten verjüngende und nach vertikal oben erweiternde Keilform 36 begrenzen, die in Figur 1c als Keil oder Trapez 38 mit gestrichelten Linien lediglich schematisch angedeutet ist. Wesentlich ist der sich nach oben erweiternde und nach unten verjüngende Aufnahmeraum, welcher durch das vordere und das hintere Stützmittel 20, 22 begrenzt wird, so dass eine stapelförmige Anordnung 4, wie in den Figuren 3a bis d und 4 dargestellt, darin aufgenommen werden kann und je nach verbleibender Dicke der stapelförmigen Anordnung 4 weiter oben oder weiter unten in vertikaler Richtung 14 in dem keilförmigen Aufnahmeraum der Haltevorrichtung in einer stabilen Stützlage positioniert wird. Erfindungsgemäß sind hierfür keine zusätzlichen Federarme oder ähnliche positionierende Mittel erforderlich, sondern die stapelförmige Anordnung 4 bzw. die Schlauchbeutelpackung 10 gelangt selbsttätig allein schwerkraftbedingt und korrelierend mit einer durch Entnahme abnehmenden Dicke der stapelförmigen Anordnung 4 weiter nach unten bis sie in einer entsprechenden Tiefe in vertikaler Richtung 14 und der Dicke der stapelförmigen Anordnung 4 ungefähr entsprechenden Breite der Keilform 36 stabil gestützt wird. Dabei wird infolge der Keilform 36 auf das hintere und das vordere Stützmittel 20, 22 ein Stützdruck im Wesentlichen quer zur Stapelebene 16 der Flachmaterialabschnitte 6 auf diese ausgeübt, wodurch der zur Entnahme anstehende Flachmaterialabschnitt 6 selbsttätig in Richtung auf die Entnahmeöffnung 12 vorgespannt wird, so dass kein leerer Hohlraum im Inneren der Schlauchbeutelpackung 10 gebildet wird. Im beispielhaft dargestellten Fall der vertikalen Orientierung des hinteren Stützmittels 22 und der schräg nach vorn geneigten Orientierung des vorderen Stützmittels 20 ist der Stützdruck mit abnehmender Dicke und zunehmender Schräglage der stapelförmigen Anordnung nicht mehr exakt quer zur Stapelebene 16 verlaufend, sondern leicht schräg hierzu, was aber im Zusammenhang mit der vorliegenden Erfindung in einem Sinn von "quer zur Stapelebene" zu verstehen ist.

Im beispielhaft dargestellten Fall schließen das vordere Stützmittel 20 und das hintere Stützmittel 22 bereichsweise einen die Keilform 36 begrenzenden Winkel α von ca 30° ein. Aufgrund einer verrundeten Ausführung des vorderen Stützmittels 20 wird dieser Winkel α nach oben hin kleiner.

Figur 4 zeigt, dass eine Breite einer erfindungsgemäß bereitzustellenden stapelförmigen Anordnung 4 eine Breite der Haltevorrichtung 2 auch durchaus übersteigen kann.

Die Anordnung des vorderen und des hinteren Stützmittels 20, 22 zueinander ist bei einer generell bevorzugten Ausführungsform der erfindungsgemäßen Haltevorrichtung wie folgt:
Im Bereich einer oberen Öffnung zum Einsetzen der stapelförmigen Anordnung 4 in die Haltevorrichtung 2 beträgt eine Tiefenerstreckung der Keilform 36, also ein Abstand 40 des vorderen Stützmittels 20 vom hinteren Stützmittel 22 in horizontaler Richtung 18, wenigstens 5 cm, insbesondere wenigstens 6 cm, insbesondere wenigstens 7 cm, insbesondere wenigstens 8 cm und insbesondere höchstens 25 cm, insbesondere höchstens 23 cm, insbesondere höchstens 20 cm, insbesondere höchstens 18 cm, insbesondere höchstens 16 cm. In vertikaler Richtung nach unten gehend verringert sich diese Tiefenerstreckung der Keilform 36, d.h. der Abstand 40 des vorderen Stützmittels 20 vom hinteren Stützmittel 22 in horizontaler Richtung 18 dann auf höchstens 8 cm, insbesondere auf höchstens 6 cm, insbesondere auf höchstens 4 cm, insbesondere auf höchstens 2 cm. Daher kann die stapelförmige Anordnung 4 schwerkraftbedingt je nach verbleibender Dicke des Stapels nach unten gleiten, so dass sie im unteren Bereich stets zwischen dem vorderen und dem hinteren Stützmittel 20, 22 unter einem moderaten schwerkraftbedingten Stützdruck lagestabilisiert ist.

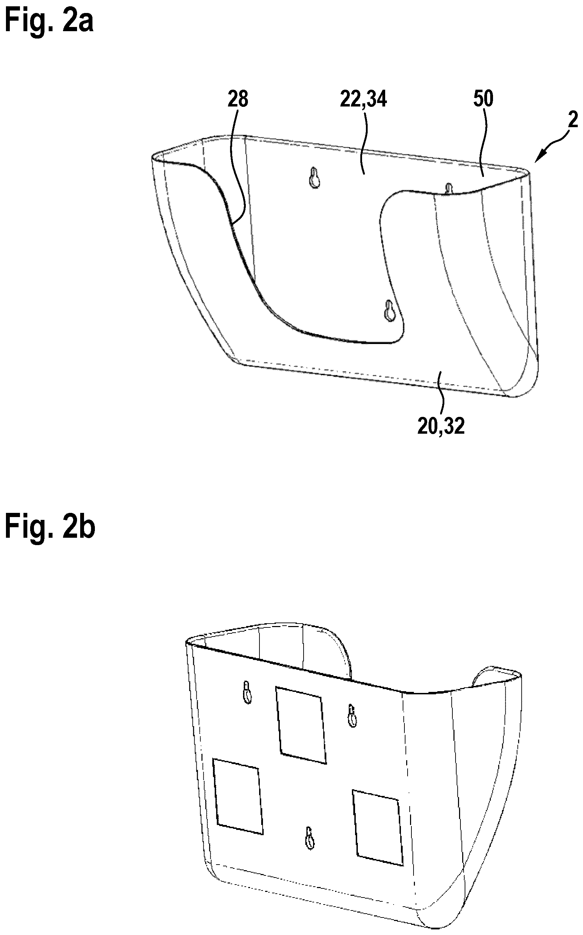

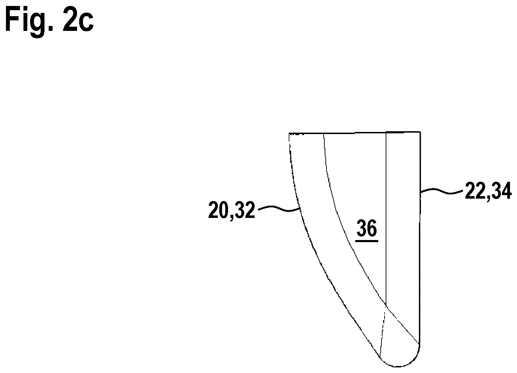

Die Figuren 2a bis c zeigen eine zweite Ausführungsform einer erfindungsgemäßen Haltevorrichtung 2 in Form eines einstückigen Kunststoffspritzgussteils 50. Dabei gehen das vordere Stützmittel 20 und das hintere Stützmittel 22 als gehäusebildende vordere und hintere Stützwände 32, 34 einstückig verrundet ineinander über, wodurch die Haltevorrichtung auch seitlich geschlossen ausgebildet ist und dadurch bis auf die vordere Ausnehmung 28 für den Zugriff auf die stapelförmige Anordnung 4 in Umfangsrichtung ein durchgehendes Gehäuse bildet, welches wie im Zusammenhang mit Figuren 1a bis c beschrieben, eine sich nach vertikal oben erweiternde und nach vertikal unten verjüngende Keilform 36 bildet.

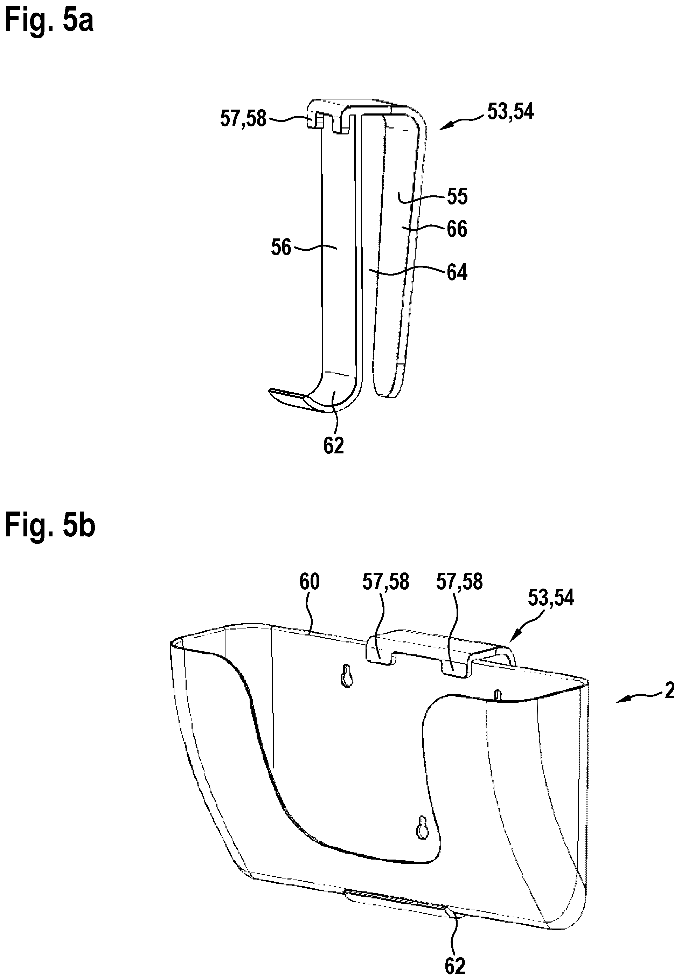

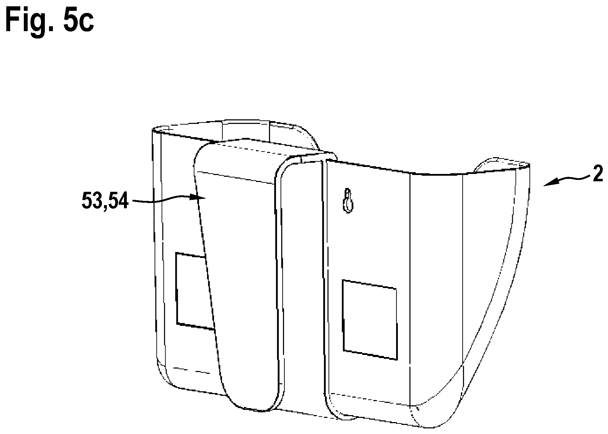

Schließlich zeigen die Figuren 5a bis c ein Montagemittel 53, welches nach Art eines Hakens 54 ausgebildet ist und einerseits formschlüssig mit der Haltevorrichtung 2 verbindbar ist und anderseits mittels eines Einhänge- oder Aufhängeabschnitts 55 über eine horizontale Stange eines Patientenbetts oder eines sonstigen übergreifbaren Elements aufhängbar ist. Das Montagemittel 53 umfasst im beispielhaft dargestellten Fall eine im Wesentlichen vertikal verlaufende Strebe 56 und oben einen Hintergriffsabschnitt 57 in Form von beispielhaft zwei nach unten abgekröpften Haltezungen 58, welche, wie in Figur 5b dargestellt, einen oberen horizontalen Randabschnitt 60 der Haltevorrichtung 2 übergreifen können. Unten umfasst das Montagemittel 54 einen beispielhaft verrundeten Stützabschnitt 62, der vorzugsweise an die Außenkontur der Haltevorrichtung 2 angepasst ist, so dass die Haltevorrichtung 2 mit ihrem unteren Ende auf diesem Stützabschnitt 62 aufsitzen kann, so wie dies in Figur 5b dargestellt ist. Der Einhänge- oder Aufhängeabschnitt 55 des Hakens 54 bildet einen nach unten öffnenden Aufnahmeschlitz 64, der einerseits von der Strebe 56 und andererseits von einer weiteren nach unten frei ausendenden Strebe 66 begrenzt ist.

## Patentansprüche

1. Vorrichtung (2) zum Halten und Bereitstellen einer insbesondere umverpackten stapelförmigen Anordnung (4) von Flachmaterialabschnitten (6), insbesondere von Reinigungs- oder Desinfektionstüchern, oder von flachgelegten Einmal-Handschuhen damit der Endverbraucher aufeinanderfolgend einzelne Flachmaterialabschnitte (6) oder Handschuhe aus der stapelförmigen Anordnung (4) entnehmen kann,
- wobei die Flachmaterialabschnitte (6) oder Handschuhe jeweils in einer Stapelebene (16) erstreckt sind und orthogonal zu dieser Stapelebene (16) mittelbar oder unmittelbar aneinander anliegen,
- wobei die Vorrichtung ein vorderes Stützmittel (20) und ein hinteres Stützmittel (22) aufweist, wobei das vordere Stützmittel (20) bei bestimmungsgemäßer Positionierung und Verwendung der Vorrichtung dem Endverbraucher zugewandt ist und eine Öffnung oder Ausnehmung (28) für den Zugriff auf die stapelförmige Anordnung (4) durch den Endverbraucher aufweist,
- wobei die Vorrichtung bei bestimmungsgemäßer Positionierung und Verwendung der Vorrichtung derart ausgebildet ist, dass die stapelförmige Anordnung (4) darin mit im Wesentlichen vertikal orientierter Stapelebene (16) aufnehmbar ist,
wobei das vordere Stützmittel (20) und das hintere Stützmittel (22) bei bestimmungsgemäßer Positionierung und Verwendung der Vorrichtung eine sich nach vertikal unten verjüngende und nach vertikal oben erweiternde Keilform (36) für die Aufnahme der insbesondere umverpackten stapelförmigen Anordnung (4) begrenzen, so dass die insbesondere umverpackte stapelförmige Anordnung (4) mit zunehmender Anzahl der bereits entnommenen Flachmaterialabschnitte (6) oder Handschuhe und damit korrelierend mit abnehmender verbleibender Dicke der stapelförmigen Anordnung (4) in der Vorrichtung schwerkraftbedingt weiter nach vertikal unten gleiten kann, so dass infolge der Keilform (36) durch das hintere und das vordere Stützmittel (20, 22) stets ein quer zur Stapelebene (16) wirkender Stützdruck auf die stapelförmige Anordnung (4) ausgeübt wird, und
wobei das vordere Stützmittel (20) und das hintere Stützmittel (22) bereichsweise einen die Keilform (36) begrenzenden Winkel (α) von wenigstens 20° einschließen.

2. Vorrichtung (2) nach Anspruch 1, **dadurch gekennzeichnet, dass** das vordere Stützmittel (20) und/oder das hintere Stützmittel (22) flächenhaft wirkend ausgebildet ist und sich über wenigstens 50 %, insbesondere über wenigstens 60 %, insbesondere über wenigstens 70 %, insbesondere über wenigstens 80 % der flächenhaften Erstreckung der stapelförmigen Anordnung in der Stapelebene und weiter insbesondere über die gesamte flächenhafte Erstreckung der stapelförmigen Anordnung in der Stapelebene erstreckt.

3. Vorrichtung (2) nach Anspruch 1 oder 2, **dadurch gekennzeichnet, dass** das vordere Stützmittel (20) und/oder das hintere Stützmittel (22) insbesondere von einem flächenhaft durchgehenden Material gebildet ist.

4. Vorrichtung (2) nach einem oder mehreren der vorstehenden Ansprüche, **dadurch gekennzeichnet, dass** das vordere Stützmittel (20) und/oder das hintere Stützmittel (22) von einer vorderen bzw. hinteren Stützwand (32, 34) gebildet sind.

5. Vorrichtung (2) nach einem oder mehreren der vorstehenden Ansprüche, **dadurch gekennzeichnet, dass** sie aus einem metallischen Flachmaterial gebogen ist.

6. Vorrichtung (2) nach einem oder mehreren der vorstehenden Ansprüche, **dadurch gekennzeichnet, dass** sie als Kunststoffspritzteil hergestellt ist.

7. Vorrichtung (2) nach einem oder mehreren der vorstehenden Ansprüche, **dadurch gekennzeichnet, dass** sie seitlich einseitig oder beidseitig offen ausgebildet ist.

8. Vorrichtung (2) nach einem oder mehreren der vorstehenden Ansprüche, **dadurch gekennzeichnet, dass** sie seitlich einseitig oder beidseitig geschlossen ausgebildet ist.

9. Vorrichtung (2) nach einem oder mehreren der vorstehenden Ansprüche, **dadurch gekennzeichnet, dass** die Öffnung oder die Ausnehmung (28) für den Zugriff auf die stapelförmige Anordnung (4) randoffen ausgebildet ist.

10. Vorrichtung (2) nach einem oder mehreren der vorstehenden Ansprüche, **dadurch gekennzeichnet, dass** kein weiteres oder zusätzliches federndes Stützmittel vorhanden ist.

11. Vorrichtung (2) nach einem oder mehreren der vorstehenden Ansprüche, **dadurch gekennzeichnet, dass** das vordere Stützmittel (20) und das hintere Stützmittel (22) ineinander übergehen, insbesondere einstückig ineinander übergehen, insbesondere verrundet ineinander übergehen.

12. Vorrichtung (2) nach einem oder mehreren der vorstehenden Ansprüche, **dadurch gekennzeichnet, dass** das vordere Stützmittel (20) bei Betrachtung einer vertikalen orthogonal zu dem vorderen und dem hinteren Stützmittel verlaufenden Schnittebene in einer Richtung von unten nach oben gekrümmt verläuft, insbesondere sich ausgehend von der Keilform einem vertikalen Verlauf annähert.

13. Vorrichtung (2) nach einem oder mehreren der vorstehenden Ansprüche, **dadurch gekennzeichnet, dass** das vordere Stützmittel (20) und das hintere Stützmittel (22) in einem oberen Bereich eine nach oben offene Einführöffnung zum Einsetzen der insbesondere umverpackten stapelförmigen Anordnung (4) in die Vorrichtung bilden.

14. Vorrichtung (2) nach einem oder mehreren der vorstehenden Ansprüche, **dadurch gekennzeichnet, dass** das vordere Stützmittel (20) und das hintere Stützmittel (22) in einem oberen Bereich, welcher eine Einführöffnung für die insbesondere umverpackte stapelförmige Anordnung bildet (4), zumindest bereichsweise parallel zueinander und bei bestimmungsgemäßer Anordnung und Verwendung vertikal orientiert sind.

15. Vorrichtung (2) nach einem oder mehreren der vorstehenden Ansprüche, **dadurch gekennzeichnet, dass** in einem oberen Bereich der Vorrichtung eine Tiefenerstreckung der Keilform (36), also ein Abstand (40) des vorderen Stützmittels (20) vom hinteren Stützmittel (22) in horizontaler Richtung (18) wenigstens 5 cm, insbesondere wenigstens 6 cm, insbesondere wenigstens 7 cm, insbesondere wenigstens 8 cm und insbesondere höchstens 25 cm, insbesondere höchstens 23 cm, insbesondere höchstens 20 cm, insbesondere höchstens 18 cm, insbesondere höchstens 16 cm beträgt und dass sich der Abstand (40) in vertikaler Richtung nach unten gehend dann auf höchstens 8 cm, insbesondere auf höchstens 6 cm, insbesondere auf höchstens 4 cm, insbesondere auf höchstens 2 cm verringert.

16. Vorrichtung (2) nach einem oder mehreren der vorstehenden Ansprüche, **dadurch gekennzeichnet, dass** das vordere Stützmittel (20) und das hintere Stützmittel (22) bereichsweise einen die Keilform (36) begrenzenden Winkel (α) von wenigstens 25°, insbesondere von wenigstens 30°, insbesondere von wenigstens 35°, insbesondere von höchstens 60°, insbesondere von höchstens 55°, insbesondere von höchstens 50°, insbesondere von höchstens 45°, insbesondere von höchstens 40° einschließen.

17. Vorrichtung (2) nach einem oder mehreren der vorstehenden Ansprüche, **dadurch gekennzeichnet, dass** das hintere Stützmittel (22) bei bestimmungsgemäßer Positionierung und Verwendung der Vorrichtung in einer vertikalen Ebene erstreckt ist.

18. Vorrichtung (2) nach einem oder mehreren der vorstehenden Ansprüche, **dadurch gekennzeichnet, dass** das hintere Stützmittel (22) ein Montagemittel (53), insbesondere ein Wandmontagemittel, aufweist, insbesondere Öffnungen (24), einen Halter oder Haken (54) oder ein Haftklebemittel (26) aufweist, insbesondere einen vorzugsweise werkzeuglos auf die Vorrichtung aufklipsbaren Halter oder Haken (54) aufweist.

19. Vorrichtung (2) nach einem oder mehreren der vorstehenden Ansprüche, **gekennzeichnet durch** einen vorzugsweise werkzeuglos auf die Vorrichtung (2) aufklipsbaren Halter oder Haken (54), welcher eine in vertikaler Ebene erstreckte Strebe (56), eine Lasche oder einen Wandabschnitt aufweist, welche(r) die Vorrichtung (2) und insbesondere das hintere Stützmittel (22) der Vorrichtung (2), von oben und von unten ergreift und hierdurch zu halten vermag.

20. Vorrichtung (2) nach Anspruch 19, **dadurch gekennzeichnet, dass** die Strebe (56), die Lasche oder der Wandabschnitt in der Gebrauchssituation oben einen Hintergriffsabschnitt (57), insbesondere einen um vorzugsweise 180° gekröpften Randabschnitt (58), aufweist, welcher einen oberen Rand des hinteren Stützmittels (22) von oben formschlüssig übergreifen oder in eine Ausnehmung des hinteren Stützmittels (22) eingreifen kann, und in der Gebrauchssituation unten einen unter die Vorrichtung (2) greifenden Stützabschnitt (62) aufweist, auf welchem die Vorrichtung (2) mit ihrem unteren Ende aufsitzen kann und hierdurch gestützt und gehalten ist.

21. Vorrichtung (2) nach Anspruch 19 oder 20, **dadurch gekennzeichnet, dass** der Halter oder Haken (54) einen Einhänge- oder Aufhängeabschnitt (55) aufweist, mittels dessen der Halter oder Haken (54) und die daran gehaltene Vorrichtung (2) samt Inhalt an einem Patientenbett oder an einem Ständer oder an einer sonstigen übergreifbaren Anordnung aufhängbar ist, und dass der Einhänge- oder Aufhängeabschnitt (55) hierfür einen nach unten öffnenden Aufnahmeschlitz (64) begrenzt, der insbesondere von der in vertikaler Ebene erstreckten Strebe (56), der Lasche oder dem Wandabschnitt einerseits begrenzt ist.

22. Vorrichtung (2) nach einem oder mehreren der vorstehenden Ansprüche, **dadurch gekennzeichnet, dass** die insbesondere umverpackte stapelförmige Anordnung (4) von Flachmaterialabschnitten (6) oder Handschuhen eine Schlauchfolien-Packung (Flowpack-Packung) (10) ist, die ihrerseits eine insbesondere wiederverschließbare Zugriffsöffnung (12) aufweist oder bei der eine solche insbesondere wiederverschließbare Zugriffsöffnung manuell durch den Endverbraucher herstellbar ist.

23. System aus einer Vorrichtung (2) nach einem oder mehreren der vorstehenden Ansprüchen und einer insbesondere umverpackten stapelförmigen Anordnung (4) von Flachmaterialabschnitten (6), insbesondere von Reinigungs- oder Desinfektionstüchern, oder von flachgelegten Einmal-Handschuhen, zur Abgabe der Flachmaterialabschnitte (6) oder Einmal-Handschuhe an Endverbraucher derart, dass ein Endverbraucher aufeinanderfolgend einzelne Flachmaterialabschnitte (6) oder Handschuhe aus der stapelförmigen Anordnung (4) entnehmen kann.

24. Verwendung der Vorrichtung (2) nach einem oder mehreren der vorstehenden Ansprüche 1-19 zum Halten und Bereitstellen einer insbesondere umverpackten stapelförmigen Anordnung (4) von Flachmaterialabschnitten (6), insbesondere von Reinigungs- oder Desinfektionstüchern, oder von flachgelegten Einmal-Handschuhen, damit ein Endverbraucher aufeinanderfolgend einzelne Flachmaterialabschnitte (6) oder Handschuhe aus der stapelförmigen Anordnung (4) entnehmen kann.

## Claims

1. Device (2) for holding and providing an in particular externally packaged stacked arrangement (4) of flat material portions (6), in particular cleaning or disinfection wipes, or of disposable gloves laid flatwise, so that the end user can successively remove individual flat material portions (6) or gloves from the stacked arrangement (4),
- wherein the flat material portions (6) or gloves are each extended in a stacking plane (16) and are directly or indirectly adjacent to one another orthogonally to this stacking plane (16),
- wherein the device has a front support means (20) and a rear support means (22), wherein the front support means (20) faces the end user when the device is positioned and used as intended and has an opening or recess (28) for the end user to access the stacked arrangement (4),
- wherein the device, when positioned and used as intended, is designed such that the stacked arrangement (4) can be received therein with a substantially vertically oriented stacking plane (16),
wherein, when the device is positioned and used as intended, the front support means (20) and the rear support means (22) limit a wedge shape (36) that tapers vertically downward and widens vertically upward for receiving the in particular externally packaged stacked arrangement (4) so that the in particular externally packaged stacked arrangement (4) can slide further vertically downward in the device due to gravity as the number of flat material portions (6) or gloves already removed increases and thus correlating with the decreasing remaining thickness of the stacked arrangement (4) so that, as a result of the wedge shape (36), a support pressure acting transversely to the stacking plane (16) is always exerted on the stacked arrangement (4) by the rear and the front support means (20, 22), and wherein the front support means (20) and the rear support means (22) form an angle (α) of at least 20° in some regions, which limits the wedge shape (36).

2. Device (2) according to claim 1, **characterized in that** the front support means (20) and/or the rear support means (22) is designed to act over a surface area and extends over at least 50%, in particular over at least 60%, in particular over at least 70%, in particular over at least 80%, of the surface-area extension of the stacked arrangement in the stacking plane and further, in particular, over the entire surface-area extension of the stacked arrangement in the stacking plane.

3. Device (2) according to claim 1 or 2, **characterized in that** the front support means (20) and/or the rear support means (22) is formed in particular from a material which is continuous across the surface area.

4. Device (2) according to one or more of the preceding claims, **characterized in that** the front support means (20) and/or the rear support means (22) is formed by a front or rear support wall (32, 34).

5. Device (2) according to one or more of the preceding claims, **characterized in that** the device is bent into shape from a metal flat material.

6. Device (2) according to one or more of the preceding claims, **characterized in that** the device is produced as a plastics molded part.

7. Device (2) according to one or more of the preceding claims, **characterized in that** the device is laterally open on one or both sides.

8. Device (2) according to one or more of the preceding claims, **characterized in that** the device is laterally closed on one or both sides.

9. Device (2) according to one or more of the preceding claims, **characterized in that** the opening or the recess (28) is open at the edges to allow access to the stacked arrangement (4).

10. Device (2) according to one or more of the preceding claims, **characterized in that** there is no further or additional spring support means.

11. Device (2) according to one or more of the preceding claims, **characterized in that** the front support means (20) and the rear support means (22) merge into one another, in particular merge into one another in one piece, in particular merge into one another in a rounded manner.

12. Device (2) according to one or more of the preceding claims, **characterized in that** when viewed in a vertical section plane orthogonal to the front and the rear support means, the front support means (20) is curved in a direction from bottom to top, in particular approaches a vertical course starting from the wedge shape.

13. Device (2) according to one or more of the preceding claims, **characterized in that** the front support means (20) and the rear support means (22) form an upwardly open insertion opening in an upper region for inserting the in particular externally packaged stacked arrangement (4) into the device.

14. Device (2) according to one or more of the preceding claims, **characterized in that** the front support means (20) and the rear support means (22), in an upper region which forms an insertion opening for the in particular externally packaged stacked arrangement (4), are, at least in some regions, parallel to one another and are vertically oriented when arranged and used as intended.

15. Device (2) according to one or more of the preceding claims, **characterized in that** in an upper region of the device, the depth extension of the wedge shape (36), i.e. the distance (40) of the front support means (20) from the rear support means (22) in the horizontal direction (18), is at least 5 cm, in particular at least 6 cm, in particular at least 7 cm, in particular at least 8 cm and in particular at most 25 cm, in particular at most 23 cm, in particular at most 20 cm, in particular at most 18 cm, in particular at most 16 cm, and **in that** the distance (40) in the vertical direction downward is then reduced to at most 8 cm, in particular to at most 6 cm, in particular to at most 4 cm, in particular to at most 2 cm.

16. Device (2) according to one or more of the preceding claims, **characterized in that** in some regions, the front support means (20) and the rear support means (22) form an angle (α) of at least 25°, in particular of at least 30°, in particular of at least 35°, in particular of at most 60°, in particular of at most 55°, in particular of at most 50°, in particular of at most 45°, in particular of at most 40°, which limits the wedge shape (36).

17. Device (2) according to one or more of the preceding claims, **characterized in that** the rear support means (22) extends in a vertical plane when the device is positioned and used as intended.

18. Device (2) according to one or more of the preceding claims, **characterized in that** the rear support means (22) has a mounting means (53), in particular a wall mounting means, in particular openings (24), a holder or hook (54) or an adhesive means (26), in particular a holder or hook (54) that can preferably be clipped onto the device without tools.

19. Device (2) according to one or more of the preceding claims, **characterized by** a holder or hook (54) which can preferably be clipped onto the device (2) without tools and which has a strut (56) extending in a vertical plane, a tab or a wall portion which grips the device (2) and in particular the rear support means (22) of the device (2) from above and from below and is, as a result, able to hold it.

20. Device (2) according to claim 19, **characterized in that** the strut (56), the tab or the wall portion in the operating situation has, at the top, a rear gripping portion (57), in particular an edge portion (58) preferably cranked by 180°, which can form-fittingly engage over an upper edge of the rear support means (22) from above or engage in a recess of the rear support means (22), and in the operating situation has, at the bottom, a support portion (62) which engages under the device (2) and on which the device (2) can sit with its lower end and is, as a result, supported and held.

21. Device (2) according to claim 19 or 20, **characterized in that** the holder or hook (54) has a suspension or hanging portion (55) by means of which the holder or hook (54) and the device (2) held thereon, including its contents, can be suspended from a patient's bed or from a stand or from any other reachable arrangement, and **in that** the suspension or hanging portion (55) for this purpose limits a downwardly opening receiving slot (64) which is in particular limited on one side by the strut (56) extending in the vertical plane, the tab or the wall portion.

22. Device (2) according to one or more of the preceding claims, **characterized in that** the in particular externally packaged stacked arrangement (4) of flat material portions (6) or gloves is a flow pack (10) which in turn has a resealable access opening (12) or in which such a resealable access opening can be manually produced by the end user.

23. System comprising a device (2) according to one or more of the preceding claims and an in particular externally packaged stacked arrangement (4) of flat material portions (6), in particular of cleaning or disinfection wipes, or of disposable gloves laid flatwise, for dispensing the flat material portions (6) or disposable gloves to end users in such a way that an end user can successively remove individual flat material portions (6) or gloves from the stacked arrangement (4).

24. Use of the device (2) according to one or more of the preceding claims 1-19 for holding and providing an in particular externally packaged stacked arrangement (4) of flat material portions (6), in particular cleaning or disinfection wipes, or of disposable gloves laid flatwise so that an end user can successively remove individual flat material portions (6) or gloves from the stacked arrangement (4).

## Revendications

1. Dispositif (2) destiné à maintenir et à fournir un agencement en forme d'empilement (4), en particulier suremballé, de sections de matériau plat (6), en particulier de lingettes nettoyantes ou désinfectantes, ou de gants jetables aplatis, afin que l'utilisateur final puisse retirer successivement des sections de matériau plat (6) individuels ou des gants individuels dudit agencement en forme d'empilement (4),
- dans lequel les sections de matériau plat (6) ou les gants s'étendent chacun dans un plan d'empilement (16) et sont directement ou indirectement adjacents les uns aux autres, orthogonalement à ce plan d'empilement (16),
- dans lequel dispositif comprend un moyen de support avant (20) et un moyen de support arrière (22), dans lequel ledit moyen de support avant (20) est orienté vers l'utilisateur final lorsque le dispositif est positionné et utilisé conformément à sa destination et présente une ouverture ou un évidement (28) permettant à l'utilisateur final d'accéder à l'agencement en forme d'empilement (4),
- dans lequel le dispositif, lorsque le dispositif est positionné et utilisé conformément à sa destination, est conçu de telle sorte que l'agencement en forme d'empilement (4) y peut être reçu de manière à ce que le plan d'empilement (16) est orienté pour l'essentiel verticalement,
dans lequel, lorsque le dispositif est positionné et utilisé conformément à sa destination, le moyen de support avant (20) et le moyen de support arrière (22) délimitent une forme de coin (36) qui se rétrécit verticalement vers le bas et s'élargit verticalement vers le haut pour recevoir l'agencement en forme d'empilement (4), en particulier suremballé, de sorte que l'agencement en forme d'empilement (4), en particulier suremballé, peut glisser davantage verticalement vers le bas dans le dispositif sous l'effet de la gravité à mesure que le nombre de sections de matériau plat (6) ou de gants déjà retirés augmente, ce qui est corrélé à la diminution de l'épaisseur restante de l'agencement en forme d'empilement (4), de sorte que, grâce à la forme de coin (36), une pression de support agissant transversalement au plan d'empilement (16) est toujours exercée sur l'agencement en forme d'empilement (4) par les moyens de support arrière et avant (20, 22), et dans lequel le moyen de support avant (20) et le moyen de support arrière (22) forment, par zones, un angle (α) d'au moins 20° qui délimite la forme de coin (36).

2. Dispositif (2) selon la revendication 1, **caractérisé par le fait que** le moyen de support avant (20) et/ou le moyen de support arrière (22) sont conçus pour agir sur une surface et s'étend(ent) sur au moins 50 %, en particulier sur au moins 60 %, en particulier sur au moins 70 %, en particulier sur au moins 80 % de l'extension surfacique de l'agencement en forme d'empilement dans le plan d'empilement, et encore en particulier sur la totalité de l'extension surfacique de l'agencement en forme d'empilement dans le plan d'empilement.

3. Dispositif (2) selon la revendication 1 ou 2, **caractérisé par le fait que** le moyen de support avant (20) et/ou le moyen de support arrière (22) sont constitués en particulier d'un matériau s'étendant en continu sur la surface.

4. Dispositif (2) selon une ou plusieurs des revendications précédentes, **caractérisé par le fait que** le moyen de support avant (20) et/ou le moyen de support arrière (22) sont respectivement constitués d'une paroi de support avant ou bien arrière (32, 34).

5. Dispositif (2) selon une ou plusieurs des revendications précédentes, **caractérisé par le fait qu'**il est plié à partir d'un matériau plat métallique.

6. Dispositif (2) selon une ou plusieurs des revendications précédentes, **caractérisé par le fait qu'**il est fabriqué en tant que pièce en matériau plastique moulée par injection.

7. Dispositif (2) selon une ou plusieurs des revendications précédentes, **caractérisé par le fait qu'**il est réalisé de manière à être ouvert latéralement sur un côté ou les deux côtés.

8. Dispositif (2) selon une ou plusieurs des revendications précédentes, **caractérisé par le fait qu'**il est réalisé de manière à être fermé latéralement sur un côté ou les deux côtés.

9. Dispositif (2) selon une ou plusieurs des revendications précédentes, **caractérisé par le fait que** l'ouverture ou l'évidement (28) est ouvert sur les bords pour permettre l'accès à l'agencement en forme d'empilement (4).

10. Dispositif (2) selon une ou plusieurs des revendications précédentes, **caractérisé par le fait qu'**aucun autre moyen de support élastique ou moyen de support élastique supplémentaire n'est présent.

11. Dispositif (2) selon une ou plusieurs des revendications précédentes, **caractérisé par le fait que** le moyen de support avant (20) et le moyen de support arrière (22) se confondent l'un l'autre, en particulier se confondent pour former une seule pièce, en particulier se confondent de manière arrondie.

12. Dispositif (2) selon une ou plusieurs des revendications précédentes, **caractérisé par le fait que** le moyen de support avant (20), lorsqu'on regarde un plan de coupe vertical s'étendant orthogonalement aux moyens de supports avant et arrière, s'étend de manière incurvée dans une direction allant de bas en haut, en particulier s'approche d'une allure verticale à partir de la forme de coin.

13. Dispositif (2) selon une ou plusieurs des revendications précédentes, **caractérisé par le fait que** le moyen de support avant (20) et le moyen de support arrière (22) forment, dans une zone supérieure, une ouverture d'insertion ouverte vers le haut permettant d'insérer ledit agencement en forme d'empilement (4), en particulier suremballé, dans le dispositif.

14. Dispositif (2) selon une ou plusieurs des revendications précédentes, **caractérisé par le fait que** le moyen de support avant (20) et le moyen de support arrière (22) sont, au moins par zones, orientés parallèlement entre eux et, lorsqu'ils sont agencés et utilisés conformément à leur destination, orientés verticalement, dans une zone supérieure qui forme une ouverture d'insertion pour l'agencement en forme d'empilement (4), en particulier suremballé.

15. Dispositif (2) selon une ou plusieurs des revendications précédentes, **caractérisé par le fait que**, dans une zone supérieure du dispositif, une extension en profondeur de la forme de coin (36), donc une distance (40) entre le moyen de support avant (20) et le moyen de support arrière (22) dans la direction horizontale (18), est d'au moins 5 cm, en particulier d'au moins 6 cm, en particulier d'au moins 7 cm, en particulier d'au moins 8 cm, et en particulier de 25 cm tout au plus, en particulier de 23 cm tout au plus, en particulier de 20 cm tout au plus, en particulier de 18 cm tout au plus, en particulier de 16 cm tout au plus, et que la distance (40) diminue ensuite, dans la direction verticale allant vers le bas, jusqu'à 8 cm tout au plus, en particulier jusqu'à 6 cm tout au plus, en particulier jusqu'à 4 cm tout au plus, en particulier jusqu'à 2 cm tout au plus.

16. Dispositif (2) selon une ou plusieurs des revendications précédentes, **caractérisé par le fait que** le moyen de support avant (20) et le moyen de support arrière (22) définissent, par zones, un angle (α) d'au moins 25°, en particulier d'au moins 30°, en particulier d'au moins 35°, en particulier d'au plus 60°, en particulier d'au plus 55°, en particulier d'au plus 50°, en particulier d'au plus 45°, en particulier d'au plus 40°, qui délimite la forme de coin (36).

17. Dispositif (2) selon une ou plusieurs des revendications précédentes, **caractérisé par le fait que** le moyen de support arrière (22) s'étend dans un plan vertical lorsque le dispositif est positionné et utilisé conformément à sa destination.

18. Dispositif (2) selon une ou plusieurs des revendications précédentes, **caractérisé par le fait que** le moyen de support arrière (22) comprend un moyen de montage (53), en particulier un moyen de montage mural, en particulier des ouvertures (24), un support ou un crochet (54), ou un moyen adhésif (26), en particulier un support ou un crochet (54) qui peut être clipsé de préférence sans outil sur le dispositif.

19. Dispositif (2) selon une ou plusieurs des revendications précédentes, **caractérisé par** un support ou un crochet (54) qui peut être clipsé sur le dispositif (2) de préférence sans outil et qui comprend une entretoise (56), une languette ou une section murale qui s'étend dans le plan vertical et qui saisit le dispositif (2), et en particulier le moyen de support arrière (22) du dispositif (2), par le haut et par le bas, et est ainsi capable de le maintenir.

20. Dispositif (2) selon la revendication 19, **caractérisé par le fait que** l'entretoise (56), la languette ou la section murale comprend en haut, en situation d'utilisation, une section d'engagement par l'arrière (57), en particulier une section de bord (58) de préférence coudé à 180°, qui peut s'engager par complémentarité de forme par le haut au-dessus d'un bord supérieur du moyen de support arrière (22) ou peut s'engager dans un évidement du moyen de support arrière (22), et comprend en bas, en situation d'utilisation, une section de support (62) qui s'engage sous le dispositif (2) et sur laquelle le dispositif (2) peut reposer par son extrémité inférieure et est ainsi supporté et maintenu.

21. Dispositif (2) selon la revendication 19 ou 20, **caractérisé par le fait que** le support ou crochet (54) comprend une section d'accrochage ou de suspension (55) au moyen de laquelle le support ou crochet (54) et le dispositif (2) qui y est maintenu, y compris son contenu, peuvent être suspendus à un lit de patient ou à un support vertical ou à tout autre dispositif apte à être enjambé, et que la section d'accrochage ou de suspension (55) délimite à cette fin une fente de réception (64) s'ouvrant vers le bas qui est en particulier délimitée d'un côté par l'entretoise (56) s'étendant dans le plan vertical, la languette ou la section murale.

22. Dispositif (2) selon une ou plusieurs des revendications précédentes, **caractérisé par le fait que** l'agencement en forme d'empilement (4), en particulier suremballé, de sections de matériau plat (6) ou de gants, est un emballage de film tubulaire (emballage flow-pack) (10) qui présente de sa part une ouverture d'accès (12), refermable en particulier, ou dans lequel une telle ouverture d'accès en particulier refermable peut être créée manuellement par l'utilisateur final.

23. Système constitué par un dispositif (2) selon une ou plusieurs des revendications précédentes et par un agencement en forme d'empilement (4), en particulier suremballé, de sections de matériau plat (6), en particulier de lingettes nettoyantes ou désinfectantes, ou de gants jetables aplatis, pour distribuer les sections de matériau plat (6) ou les gants jetables à des utilisateurs finaux de telle sorte qu'un utilisateur final puisse retirer successivement des sections de matériau plates (6) individuels ou des gants individuels dudit agencement en forme d'empilement (4).

24. Utilisation du dispositif (2) selon une ou plusieurs des revendications précédentes 1 à 19, pour maintenir et fournir un agencement en forme d'empilement (4), en particulier suremballé, de sections de matériau plat (6), en particulier de lingettes nettoyantes ou désinfectantes, ou de gants jetables aplatis, afin qu'un utilisateur final puisse retirer successivement des sections de matériau plat (6) individuels ou des gants individuels dudit agencement en forme d'empilement (4).
